Europäisches Patentamt

European Patent Office

Office européen des brevets

(11) Veröffentlichungsnummer: **0 071 038**
**B1**

(12) **EUROPÄISCHE PATENTSCHRIFT**

(45) Veröffentlichungstag der Patentschrift:
06.06.84

(51) Int. Cl.³: **C 07 C 79/12**

(21) Anmeldenummer: **82105968.0**

(22) Anmeldetag: **05.07.82**

(54) **Verfahren zur Herstellung von 2-Chlor-4-nitrotoluol.**

(30) Priorität: **18.07.81 DE 3128442**

(43) Veröffentlichungstag der Anmeldung:
**09.02.83 Patentblatt 83/6**

(45) Bekanntmachung des Hinweises auf die Patenterteilung:
**06.06.84 Patentblatt 84/23**

(84) Benannte Vertragsstaaten:
**CH DE FR GB LI**

(56) Entgegenhaltungen:
**DE - A - 2 021 197**
**US - A - 3 005 031**

**CHEMICAL ABSTRACTS, Band 83, Nr. 13, 29. September 1975, Seite 511, Nr. 113927q, Columbus Ohio (USA)**

(73) Patentinhaber: **BAYER AG, Konzernverwaltung RP Patentabteilung, D-5090 Leverkusen 1 Bayerwerk (DE)**

(72) Erfinder: **Petruck, Gerd-Michael, Dr., Doerpfeldstrasse 49, D-4006 Erkrath (DE)**
Erfinder: **Wambach, Paul Reimund, Dr., Bertha-von-Suttner-Strasse 65, D-5090 Leverkusen 1 (DE)**
Erfinder: **Wissner, Adolf, Dr., Am Wasserturm 13, D-5090 Leverkusen 3 (DE)**

## Beschreibung

Die Erfindung betrifft ein Verfahren zur Herstellung von 2-Chlor-4-nitrotoluol durch Chlorierung von 4-Nitrotoluol.

Es ist bekannt, 4-Nitrotoluol in Gegenwart von wasserfreiem Eisen-(III)-chlorid (J. Chem.-Soc. 1927, 2903) und Antimon-(V)-chlorid (Bull. Soc.-.chim. belges 61, 317 (1952)), weiterhin in Gegenwart von Jod in Kombination mit Eisen (Verhältnis 1 : 10) (Naturwiss. 17, 13 (1929) und Houben-Weyl, Methoden der organischen Chemie, Band V/3, 704 (1962)) sowie in Gegenwart von Eisen-(III)-chlorid und Jod (JP-AS 75/7589) bzw. in Gegenwart von Eisen, Jod und Phosphortrichlorid (US-PS 3 005 031) zu chlorieren.

Nachteilig bei diesen Verfahren ist, daß unerwünschte Nebenprodukte in erheblichem Maße gebildet werden und das gewünschte 2-Chlor-4-nitrotoluol nur in unbefriedigenden Mengen entsteht. Zur Abtrennung und Isolierung des 2-Chlor-4-nitrotoluols von den Nebenprodukten sind außerdem aufwendige Trennoperationen erforderlich, was die Wirtschaftlichkeit der genannten Verfahren stark beeinträchtigt. Zum Beispiel erhält man bei der Chlorierung von 4-Nitrotoluol in Gegenwart von Eisen und Jod (Gewichtsverhältnis 10 : 1) das gewünschte 2-Chlor-4-nitrotoluol nur zu maximal 95,1%, während in erheblichen Mengen, nämlich zu wenigstens 2,6%, unerwünschte Nebenprodukte gebildet werden (siehe Vergleichsbeispiel).

Es wurde nun ein Verfahren zur Herstellung von 2-Chlor-4-nitrotoluol durch Chlorierung von 4-Nitrotoluol gefunden, das dadurch gekennzeichnet ist, daß man die Chlorierung bei Temperaturen vom Schmelzpunkt des eingesetzten Produkts bis 120° C und 0,6 bis 1,2 Mol Chlor pro Mol eingesetztes Produkt in Gegenwart von 0,1 bis 10 Gew.-% Jod, bezogen auf eingesetztes Produkt, durchführt.

Bei dem erfindungsgemäßen Verfahren kann sowohl reines 4-Nitrotoluol als auch technisches 4-Nitrotoluol eingesetzt werden.

Bevorzugt werden in das erfindungsgemäße Verfahren 0,7 bis 1,05 Mol Chlor, besonders bevorzugt 0,9 bis 1,0 Mol Chlor, pro Mol eingesetztes Produkt eingesetzt.

Die erfindungsgemäße Umsetzung wird vorzugsweise in Gegenwart von 0,3 bis 1 Gew.-% Jod, bezogen auf eingesetztes Produkt, durchgeführt. Bevorzugt wird das kostengünstige technisch reine Jod eingesetzt.

Das erfindungsgemäße Verfahren wird bevorzugt bei 50 bis 100° C, besonders bevorzugt bei 60 bis 80° C, durchgeführt.

Das erfindungsgemäße Verfahren kann mit oder ohne Lösungsmittel durchgeführt werden. Als Lösungsmittel können die bei der Kernchlorierung üblichen Lösungsmittel eingesetzt werden, beispielsweise chlorierte Kohlenwasserstoffe, wie Methylenchlorid, Tetrachlorkohlenstoff, Ethylenchlorid, Tetrachlorethan, Trichlorbenzole und/oder Tetrachlorbenzole. Bevorzugt wird ohne Lösungsmittel chloriert.

Das erfindungsgemäße Verfahren kann bei Normaldruck oder erhöhtem Druck, z. B. bei 1 bis 10 bar, durchgeführt werden. Vorzugsweise wird bei Normaldruck gearbeitet.

Das erfindungsgemäße Verfahren kann kontinuierlich oder diskontinuierlich durchgeführt werden.

Das erfindungsgemäße Verfahren kann so durchgeführt werden, daß man zu vorgelegtem 4-Nitrotoluol und Jod Chlor hinzugibt oder zu dem vorgelegten 4-Nitrotoluol gleichzeitig eine Lösung von Jod oder Chlor eindosiert. Dabei kann Jod und Chlor in den oben erwähnten organischen Lösungsmitteln gelöst werden.

Eine bevorzugte Ausführungsform des erfindungsgemäßen Verfahrens sei im folgenden dargelegt:

In eine Mischung aus 4-Nitrotoluol und Jod werden 0,6 bis 1,2 Mol Chlor pro Mol 4-Nitrotoluol eingeleitet. In bevorzugter Weise wird gasförmiges Chlor in das Gemisch eingeleitet.

Anschließend wird das Jod aus dem Reaktionsgemisch mit Wasser oder besonders vorteilhaft mit einer Kalijodid- oder Natriumbisulfitlösung herausgewaschen. Man erhält ein Produkt, das einen besonders hohen Anteil an 2-Chlor-4-nitrotoluol (über 98%) neben nur einem geringen Anteil an Nebenprodukten (weniger als 1,4%) enthält.

Dies ist umso überraschender, als aus J. Chem. Soc. 121, 806 (1922) bekannt ist, daß bei der Chlorierung von 2-Chlor-4-nitrotoluol zu 2,6-Dichlor-4-nitrotoluol in Gegenwart von Jod ein besonders hoher Anteil seitenkettenchlorierter Nebenprodukte gebildet wird.

2-Chlor-4-nitrotoluol ist ein Zwischenprodukt für das Herbizid Chlortoluron (Pesticide Manual des Brit. Crop. Protection Council; belgisches Patent 728 267).

Die nachfolgenden Beispiele sollen das erfindungsgemäße Verfahren verdeutlichen.

### Beispiel 1

6850 g (49,9 Mol) 4-Nitrotoluol werden in Gegenwart von 34,2 g (0,5 Gew.-%) Jod bei 60° C bis zu einer Dichte $D_4^{60}$ von 1,29 g/cm³ chloriert. Es werden etwa 1 Mol Chlor pro Mol 4-Nitrotoluol umgesetzt. Die gaschromatographische Analyse ergibt:

| | |
|---|---|
| 98,1% | 2-Chlor-4-nitrotoluol |
| 1,4% | 4-Nitrotoluol |
| 0,5% | Nebenprodukte |

Ausbeute: 8423 g (49,1 Mol; 98,3%, bezogen auf das eingesetzte 4-Nitrotoluol).

### Beispiel 2

685 g (5 Mol) 4-Nitrotoluol werden in Gegenwart von 6,9 g (1 Gew.-%) Jod im Glaskolben bei 80°C chloriert bis zu einer Dichte von ca. 1,280 (bei Reaktionstemperatur). Dabei wurden etwa 1 Mol Chlor pro Mol 4-Nitrotoluol umgesetzt.

Die gaschromatographische Analyse ergibt:

| | |
|---|---|
| 98,2% | 2-Chlor-4-nitrotoluol |
| 0,4% | 4-Nitrotoluol |
| 1,4% | Nebenprodukte |

Ausbeute: 848 g (4,94 Mol; 98,8%, bezogen auf das eingesetzte 4-Nitrotoluol)

Beispiel 3

731 g (5,33 Mol) 4-Nitrotoluol werden in Gegenwart von 7,3 g (1 Gew.-%) Jod in einer Glasblasensäule (Füllhöhe (begast) 110 cm; Durchmesser 3 cm) bei 70 bis 80°C chloriert. Bei einer Dichte von $D_4^{70}$ von ca. 1,295 g/cm³ ergab die gaschromatographische Analyse:

| | |
|---|---|
| 97,4% | 2-Chlor-4-nitrotoluol |
| 1,4% | 4-Nitrotoluol |
| 1,2% | Nebenprodukte |

Ausbeute: 906 g (5,28 Mol; 99,1%, bezogen auf das eingesetzte 4-Nitrotoluol).

Beispiel 4
(Vergleichsbeispiel mit FeCl₃/J₂)

1370 g (10 Mol) 4-Nitrotoluol werden in Gegenwart von 13,7 g (1,0 Gew.-%) Eisen-III-chlorid und 1,4 g 0,1 Gew.-%) Jod bei 70°C chloriert. Bei einer Dichte $D_4^{70}$ von 1,287 g/cm³ ergab die gaschromatographische Analyse:

| | |
|---|---|
| 95,1% | 2-Chlor-4-nitrotoluol |
| 2,3% | 4-Nitrotoluol |
| 2,6% | Nebenprodukte |

Ausbeute: 1,693 g (9,87 mol; 98,7% Rohprodukt bezogen auf das eingesetzte 4-Nitrotoluol).

Patentansprüche

1. Verfahren zur Herstellung von 2-Chlor-4-nitrotoluol durch Chlorierung von 4-Nitrotoluol, dadurch gekennzeichnet, daß man die Chlorierung bei Temperaturen vom Schmelzpunkt des eingesetzten Produktes bis 120°C mit 0,6 bis 1,2 Mol Chlor pro Mol eingesetztes Produkt in Gegenwart von 0,1 bis 10 Gew.-% Jod, bezogen auf eingesetztes Produkt, durchführt.

2. Verfahren nach Anspruch 1, dadurch gekennzeichnet, daß man 0,7 bis 1,05 Mol Chlor pro Mol eingesetztes Produkt einsetzt.

3. Verfahren nach Anspruch 1, dadurch gekennzeichnet, daß man 0,9 bis 1,0 Mol Chlor pro Mol eingesetztes Produkt einsetzt.

4. Verfahren nach Ansprüchen 1 bis 3, dadurch gekennzeichnet, daß man die Chlorierung in Gegenwart von 0,3 bis 1,0 Gew.-% Jod, bezogen auf eingesetztes Produkt, durchführt.

5. Verfahren nach Ansprüchen 1 bis 4, dadurch gekennzeichnet, daß man die Umsetzung bei Temperaturen von 50 bis 100°C durchführt.

6. Verfahren nach Ansprüchen 1 bis 4, dadurch gekennzeichnet, daß man die Umsetzung bei Temperaturen von 60 bis 80°C durchführt.

7. Verfahren nach Ansprüchen 1 bis 6, dadurch gekennzeichnet, daß man die Umsetzung in Gegenwart von Methylenchlorid, Tetrachlorkohlenstoff, Ethylenchlorid, Tetrachlorethan, Trichlorbenzolen und/oder Tetrachlorbenzolen durchführt.

**Claims**

1. Process for the preparation of 2-chloro-4-nitro-toluene by chlorination of 4-nitrotoluene, characterized in that the chlorination is carried out at temperatures from the melting point of the product employed up to 120°C with 0.6 to 1.2 mol of chlorine per mol of product employed in the presence of 0.1 to 10% by weight of iodine relative to the product employed.

2. Process according to claim 1, characterized in that 0.7 to 1.05 mol of chlorine is employed per mol of product employed.

3. Process according to claim 1, characterized in that 0.9 to 1.0 mol of chlorine is employed per mol of product employed.

4. Process according to claims 1 to 3, characterized in that the chlorination is carried out in the presence of 0.3 to 1.0% by weight of iodine relative to product employed.

5. Process according to claims 1 to 4, characterized in that the reaction is carried out at temperatures from 50 to 100°C.

6. Process according to claims 1 to 4, characterized in that the reaction is carried out at temperatures from 60 to 80°C.

7. Process according to claims 1 to 6, characterized in that the reaction is carried out in the presence of methylene chloride, carbon tetrachloride, ethylene chloride, tetrachloroethane, trichlorobenzenes and/or tetrachlorobenzenes.

**Revendications**

1. Procédé de production de 2-chloro-4-nitrotoluène par chloration de 4-nitrotoluène, caractérisé en ce qu'on conduit la chloration à des températures allant du point de fusion du produit mis en oeuvre jusqu'à 120°C, avec 0,6 à 1,2 mole de chlore par mole du produit mis en oeuvre, en présence de 0,1 à 10% en poids d'iode par rapport au produit mis en oeuvre.

2. Procédé suivant la revendication 1, caractérisé en ce qu'on utilise 0,7 à 1,05 mole de chlore par mole de produit mis en oeuvre.

3. Procédé suivant la revendication 1, caractérisé en ce qu'on utilise 0,9 à 1,0 mole de chlore par mole de produit mis en oeuvre.

4. Procédé suivant les revendications 1 à 3, caractérisé en ce qu'on conduit la chloration en présence de 0,3 à 1,0% en poids d'iode par rapport au produit mis en oeuvre.

5. Procédé suivant les revendications 1 à 4, caractérisé en ce qu'on conduit la réaction à des températures de 50 à 100°C.

6. Procédé suivant les revendications 1 à 4, caractérisé en ce qu'on conduit la réaction à des températures de 60 à 80°C.

7. Procédé suivant les revendications 1 à 6, caractérisé en ce qu'on conduit la réaction en présence de chlorure de méthylène, de tétrachlorure de carbone, de chlorure d'éthylène, de tétrachloréthane, de trichlorobenzènes et/ou de tétrachlorobenzènes.